(19) Europäisches Patentamt
European Patent Office
Office européen des brevets

(11) **EP 2 142 210 B1**

(12) **EUROPEAN PATENT SPECIFICATION**

(45) Date of publication and mention
of the grant of the patent:
**24.08.2016 Bulletin 2016/34**

(51) Int Cl.:
*A61K 39/00* (2006.01)     *C07K 16/28* (2006.01)

(21) Application number: **08750865.1**

(22) Date of filing: **30.04.2008**

(86) International application number:
**PCT/IB2008/001072**

(87) International publication number:
**WO 2008/132601 (06.11.2008 Gazette 2008/45)**

(54) **CYTOTOXIC ANTI-LAG-3 MONOCLONAL ANTIBODY AND ITS USE IN THE TREATMENT OR PREVENTION OF ORGAN TRANSPLANT REJECTION AND AUTOIMMUNE DISEASE**

ZYTOTOXISCHER MONOKLONALER ANTI-LAG-3-ANTIKÖRPER UND SEINE VERWENDUNG BEI DER BEHANDLUNG UND VORBEUGUNG VON ORGANTRANSPLANTATABSTOSSUNG UND AUTOIMMUNERKRANKUNGEN

ANTICORPS MONOCLONAL ANTI-LAG-3 CYTOTOXIQUE ET SON UTILISATION DANS LE TRAITEMENT OU LA PRÉVENTION D'UN REJET DU GREFFON D'ORGANE ET DE MALADIES AUTO-IMMUNES

(84) Designated Contracting States:
**AT BE BG CH CY CZ DE DK EE ES FI FR GB GR HR HU IE IS IT LI LT LU LV MC MT NL NO PL PT RO SE SI SK TR**

(30) Priority: **30.04.2007 EP 07290545**

(43) Date of publication of application:
**13.01.2010 Bulletin 2010/02**

(73) Proprietors:
- **Immutep**
  **91893 Orsay Cedex (FR)**
- **INSERM (Institut National de la Santé et de la Recherche Médicale)**
  **75654 Paris Cedex 13 (FR)**

(72) Inventors:
- **TRIEBEL, Frédéric**
  **F-78000 Versailles (FR)**
- **VANHOVE, Bernard**
  **F-44400 Rezé (FR)**
- **HAUDEBOURG, Thomas**
  **F-44000 Nantes (FR)**

(74) Representative: **Lee, Alison Lesley**
**GlaxoSmithKline**
**Global Patents (CN925.1)**
**980 Great West Road**
**Brentford, Middlesex TW8 9GS (GB)**

(56) References cited:
**WO-A-2004/078928     WO-A1-2005/103086**
**US-A- 5 976 877**

- **MACON-LEMAITRE LAETITIA ET AL: "The negative regulatory function of the lymphocyte-activation gene-3 coreceptor (CD223) on human T cells" IMMUNOLOGY, vol. 115, no. 2, June 2005 (2005-06), pages 170-178, XP002453818 ISSN: 0019-2805**
- **MONK NICOLA J ET AL: "Fc-dependent depletion of activated T cells occurs through CD40L-specific antibody rather than costimulation blockade." NATURE MEDICINE, vol. 9, no. 10, October 2003 (2003-10), pages 1275-1280, XP002453819 ISSN: 1078-8956**
- **HAUDEBOURG THOMAS ET AL: "Depletion of LAG-3 positive cells in cardiac allograft reveals their role in rejection and tolerance" TRANSPLANTATION (HAGERSTOWN), vol. 84, no. 11, December 2007 (2007-12), pages 1500-1506, XP002495209 ISSN: 0041-1337**
- **MALGORZATA KISIELOW ET AL: "Expression of lymphocyte activation gene 3 (LAG-3) on B cells is induced by T cells", EUROPEAN JOURNAL OF IMMUNOLOGY, vol. 35, no. 7, 1 July 2005 (2005-07-01), pages 2081-2088, XP055162755, ISSN: 0014-2980, DOI: 10.1002/eji.200526090**

- Elizabeth S Song ET AL: "Rat and Human Natural Killers Exhibit Contrasting Immunoglobulin G Subclass Specificities in Anti body-Dependent Cellular Cytotoxicity Reflecting Differences in Their Fc Receptors (FcyR)", Journal of Leukocyte Biology, vol. 48, 1 January 1990 (1990-01-01), pages 524-530, XP055162883,
- Roseanna E.G. Hargreaves ET AL: "Selective depletion of activated T cells: the CD40L-specific antibody experience", Molecular medicine today, vol. 10, no. 3, 1 March 2004 (2004-03-01), pages 130-135, XP055055489, ISSN: 1471-4914, DOI: 10.1016/j.molmed.2004.01.009
- Isaacs: "From bench to bedside: discovering rules for antibody design, and improving serotherapy with monoclonal antibodies.", Rheumatology, vol. 40, no. 7, 1 July 2001 (2001-07-01), pages 724-738, XP055055488, ISSN: 1462-0324
- BERTRAND HUARD ET AL: "LAG-3 does not define a specific mode of natural killing in human", IMMUNOLOGY LETTERS, ELSEVIER BV, NL, vol. 61, 1 January 1998 (1998-01-01), pages 109-112, XP009138517, ISSN: 0165-2478
- DRAKE C G ET AL: "Blocking the regulatory T cell molecule LAG-3 augments in vivo anti-tumor immunity in an autochthonous model of prostate cancer", JOURNAL OF CLINICAL ONCOLOGY, AMERICAN SOCIETY OF CLINICAL ONCOLOGY, US, vol. 24, no. 18, suppl, 20 June 2006 (2006-06-20), page 2573, XP009138507, ISSN: 0732-183X
- HUANG CHING-TAI ET AL: "Role of LAG-3 in regulatory T cells", IMMUNITY, CELL PRESS, US, vol. 21, no. 4, 1 October 2004 (2004-10-01), pages 503-513, XP009138514, ISSN: 1074-7613, DOI: 10.1016/J.IMMUNI.2004.08.010 [retrieved on 2004-10-19]
- HUARD B ET AL: "LYMPHOCYTE-ACTIVATION GENE 3/MAJOR HISTOCOMPATIBILITY COMPLEX CLASS II INTERACTION MODULATES THE ANTIGENIC RESPONSE OF CD4+ T LYMPHOCYTES", EUROPEAN JOURNAL OF IMMUNOLOGY, WILEY - V C H VERLAG GMBH & CO. KGAA, DE, vol. 24, no. 12, 1 January 1994 (1994-01-01), pages 3216-3221, XP000672156, ISSN: 0014-2980, DOI: 10.1002/EJI.1830241246
- YOUSAF N ET AL: "TARGETING BEHAVIOR OF RAT MONOCLONAL IGG ANTIBODIES IN-VIVO ROLE OF ANTIBODY ISOTYPE SPECIFICITY AND THE TARGET CELL ANTIGEN DENSITY", EUROPEAN JOURNAL OF IMMUNOLOGY, WILEY - V C H VERLAG GMBH & CO. KGAA, DE, vol. 21, no. 4, 1 January 1991 (1991-01-01), pages 943-950, XP009138554, ISSN: 0014-2980, DOI: 10.1002/EJI.1830210413
- CHAN L A ET AL: "Variable region domain exchange in human IgGs promotes antibody complex formation with accompanying structural changes and altered effector functions", MOLECULAR IMMUNOLOGY, PERGAMON, GB, vol. 41, no. 5, 1 July 2004 (2004-07-01), pages 527-538, XP002519620, ISSN: 0161-5890, DOI: 10.1016/J.MOLIMM.2004.03.034
- NISHIDA MICHIO ET AL: "Novel humanized anti-CD20 monoclonal antibodies with unique germline VH and VL gene recruitment and potent effector functions", INTERNATIONAL JOURNAL OF ONCOLOGY, DEMETRIOS A. SPANDIDOS ED. & PUB, GR, vol. 32, no. 6, 1 June 2008 (2008-06-01), pages 1263-1274, XP009138553, ISSN: 1019-6439, DOI: 10.3892/IJO_32_6_1263
- TEELING JESSICA L ET AL: "The biological activity of human CD20 monoclonal antibodies is linked to unique epitopes on CD20", THE JOURNAL OF IMMUNOLOGY, THE AMERICAN ASSOCIATION OF IMMUNOLOGISTS, US, vol. 177, no. 1, 1 July 2006 (2006-07-01), pages 362-371, XP002453762, ISSN: 0022-1767
- BINDON C I ET AL: "IMPORTANCE OF ANTIGEN SPECIFICITY FOR COMPLEMENT-MEDIATED LYSIS BY MONOCLONAL ANTIBODIES", EUROPEAN JOURNAL OF IMMUNOLOGY, WILEY - V C H VERLAG GMBH & CO. KGAA, DE, vol. 18, no. 10, 1 January 1988 (1988-01-01), pages 1507-1514, XP009138552, ISSN: 0014-2980, DOI: 10.1002/EJI.1830181006
- IYAD JAMALI ET AL: "KINETICS OF ANTI-CD4-INDUCED T HELPER CELL DEPLETION AND INHIBITION OF FUNCTION Activation of T Cells by the CD3 Pathway Inhibits Anti-CD4-Mediated T Cell Elimination and Down-Regulation of Cell Surface CD4", THE JOURNAL OF IMMUNOLOGY, vol. 148, no. 6, 15 March 1992 (1992-03-15), pages 1613-1619, XP055213914, ISSN: 0022-1767
- MONK NICOLA J ET AL: "Fc-dependent depletion of activated T cells occurs through CD40L-specific antibody rather than costimulation blockade", NATURE MEDICINE, NATURE PUBLISHING GROUP, NEW YORK, NY, US, vol. 9, no. 10, 1 October 2003 (2003-10-01), pages 1275-1280, XP002453819, ISSN: 1078-8956, DOI: 10.1038/NM931
- N. POIRIER ET AL: "Antibody-mediated depletion of lymphocyte-activation gene-3 (LAG-3+)-activated T lymphocytes prevents delayed-type hypersensitivity in non-human primates", CLINICAL & EXPERIMENTAL IMMUNOLOGY, vol. 164, no. 2, 1 May 2011 (2011-05-01), pages 265-274, XP055055512, ISSN: 0009-9104, DOI: 10.1111/j.1365-2249.2011.04329.x

- TRIEBEL F ET AL: "LAG-3 A NOVEL LYMPHOCYTE ACTIVATION GENE CLOSELY RELATED TO CD4", THE JOURNAL OF EXPERIMENTAL MEDICINE, ROCKEFELLER UNIVERSITY PRESS, US, vol. 171, no. 5, 1 May 1990 (1990-05-01), pages 1393-1405, XP000672284, ISSN: 0022-1007, DOI: 10.1084/JEM.171.5.1393
- BAIXERAS E ET AL: "CHARACTERIZATION OF THE LYMPHOCYTE ACTIVATION GENE 3-ENCODED PROTEIN. A NEW LIGAND FOR HUMAN LEUKOCYTE ANTIGEN CLASS II ANTIGENS", THE JOURNAL OF EXPERIMENTAL MEDICINE, ROCKEFELLER UNIVERSITY PRESS, US, vol. 176, 1 August 1992 (1992-08-01), pages 327-337, XP000677772, ISSN: 0022-1007, DOI: 10.1084/JEM.176.2.327
- HUARD B ET AL: "CELLULAR EXPRESSION AND TISSUE DISTRIBUTION OF THE HUMAN LAG-3-ENCODED PROTEIN, AN MHC CLASS II LIGAND", IMMUNOGENETICS, SPRINGER VERLAG, BERLIN, DE, vol. 39, no. 3, 1 January 1994 (1994-01-01), pages 213-217, XP000672151, ISSN: 0093-7711, DOI: 10.1007/BF00241263
- VELDERS M P ET AL: "THE IMPACT OF ANTIGEN DENSITY AND ANTIBODY AFFINITY ON ANTIBODY-DEPENDENT CELLULAR CYTOTOXICITY: RELEVANCE FOR IMMUNOTHERAPY OF CARCINOMAS", BRITISH JOURNAL OF CANCER, NATURE PUBLISHING GROUP, GB, vol. 78, no. 4, 1 August 1998 (1998-08-01) , pages 478-483, XP009027871, ISSN: 0007-0920
- UCHIDA J ET AL: "The innate mononuclear phagocyte network depletes B lymphocytes through Fc receptor-dependent mechanisms during anti-CD20 antibody immunotherapy", THE JOURNAL OF EXPERIMENTAL MEDICINE, ROCKEFELLER UNIVERSITY PRESS, US, vol. 199, no. 12, 21 June 2004 (2004-06-21), pages 1659-1669, XP003002436, ISSN: 0022-1007, DOI: 10.1084/JEM.20040119

**Description**

**I - Field of the invention**

The present invention is in the field of immunotherapy. More specifically, it relates to the treatment or prevention of organ transplant rejection or for treating autoimmune disease. The invention relates to cytotoxic anti-LAG-3 antibodies and fragments thereof binding to LAG-3 protein and causing depletion of LAG-3$^+$ activated T cells.

**II - Background**

Lymphocyte activation gene-3 (LAG-3, CD223) is up-regulated during the early stages of T-cell activation. The present invention is based on the analysis of the effects of cytotoxic antibodies against LAG-3 in acute cardiac allograft rejection *(in vivo* animal studies) and in *in vitro* experiments where selected LAG-3 monoclonal antibodies are efficient at low doses (<0.1 μg/ml) at depleting LAG-3$^+$ activated effector T cells.

Selectively depleting activated T lymphocytes might represent an immunosuppressive induction treatment able to result in the development of regulatory cells supporting a long-term survival of allogeneic organs in mice and primates (*1*). This has actually been demonstrated with anti-CD40L antibodies that deplete in vivo activated T cells through a Fc-dependent mechanism (*2*). However, anti-CD40L antibodies also target activated platelets in humans and affect the stability of arterial thrombi (*3*). Therefore the development of monoclonal antibodies to other molecules specific for T-cell activation has catalyzed attempts to achieve immunosuppression. One such molecule is LAG-3, which engages Class II on dendritic cells (DC) with a high affinity, enabling DC to become activated (*4-6*). The LAG-3 protein is expressed *in vivo* in activated CD4$^+$ and CD8$^+$ lymphocytes residing in inflamed secondary lymphoid organs or tissues but not in spleen, thymus or blood (*7*). In addition, LAG-3 can function as a negative regulator of activated human CD4 and CD8 T cells by inhibiting early events in primary activation (*8*).

WO 2004/078928A discloses methods for the enhancement of the regulatory T-cell response, for example by increasing the number of CD223$^+$ cells in a T-cell population.

WO 2005/103086A1 discloses methods for the treatment of T cell mediated conditions by depletion of ICOS-positive cells *in vivo.*

Malgorzata et al, European Journal of Ummunology, vol. 35, no. 7, 1 July 2005, pages 2081-2088 discloses that expression of LAG-3 on B cells is induced by T cells.

**III** - **Summary of the invention**

In a first embodiment, the present invention provides a cytotoxic anti-LAG-3 monoclonal antibody or fragment thereof capable of causing depletion of LAG-3$^+$ activated T cells, wherein said antibody is produced by the hybridoma deposited at the CNCM on April 27, 2007 under the accession number CNCM I-3755, and further wherein said antibody or fragment thereof exhibits complement-dependent cytotoxicity (CDC) and/or antibody-dependent cell cytotoxicity (ADCC).

In a second embodiment, the present invention provides a cytotoxic anti-LAG-3 monoclonal antibody or fragment thereof capable of causing depletion of LAG-3$^+$ activated T cells, wherein said antibody is produced by the hybridoma deposited at the CNCM on April 27, 2007 under the accession number CNCM I-3756, and further wherein said antibody or fragment thereof exhibits complement-dependent cytotoxicity (CDC) and/or antibody-dependent cell cytotoxicity (ADCC).

Antibodies and fragments thereof according to the present invention bind to LAG-3 protein and cause depletion of LAG-3$^+$ activated T cells. Said depletion can be measured by changes in peripheral blood lymphocyte numbers, in a tissue or an organ.

The present invention further provides a cytotoxic anti-LAG-3 monoclonal antibody or fragment thereof according to the present invention for use in treating or preventing organ transplant rejection or for use in treating a T-cell mediated autoimmune disease.

In a further embodiment, the present invention provides a pharmaceutical composition comprising from 30 to 300 mg per dose of a cytotoxic anti-LAG-3 monoclonal antibody of the present invention, and a pharmaceutically acceptable carrier and/or diluent for administration to a mammal.

**IV - Description of the figures**

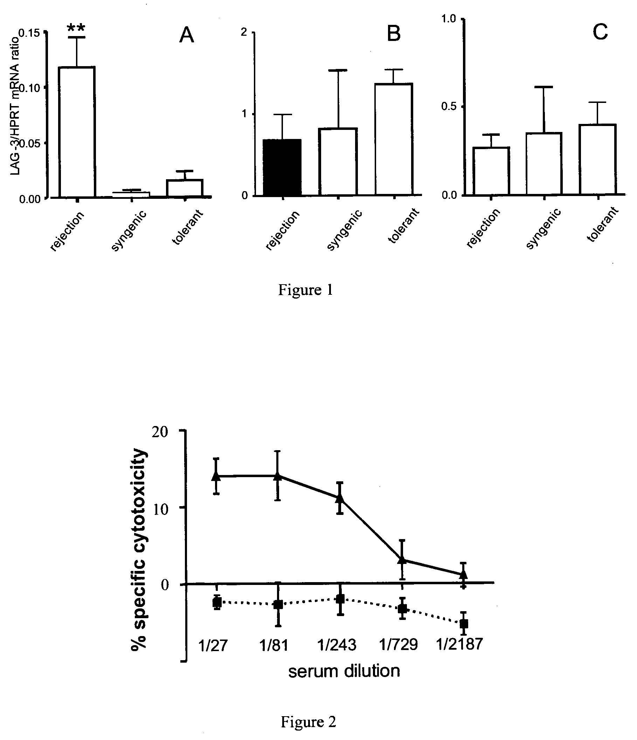

Figure 1: LAG-3 mRNA expression in cardiac allograft (A), in the spleen (B) and in lymph nodes (C). Expression of LAG-3 mRNA in heart grafts at day 5 was measured by quantitative RT-PCR and compared with housekeeping HPRT transcripts expression. Rejection: allograft without treatment. Syngenic: isograft. Tolerant: allograft in recipients receving a tolerogenic (CsA+anti-CD28 antibodies) treatment. **: $p < 0.05$ for syngenic and tolerant vs. rejection.

Figure 2: Characterization of anti-LAG-3 antibodies in a complement-dependant cytotoxicity assay. ConA-stimulated target cells were labeled with $^{51}$Cr and mixed with rabbit complement and anti-LAG-3 (full line) or preimmune (dotted line) serum at the indicated dilution. % cytotoxic activity is calculated as follows: (CPM of the assay - spontaneous CPM release)/ maximum released CPM obtained after cell lysis.

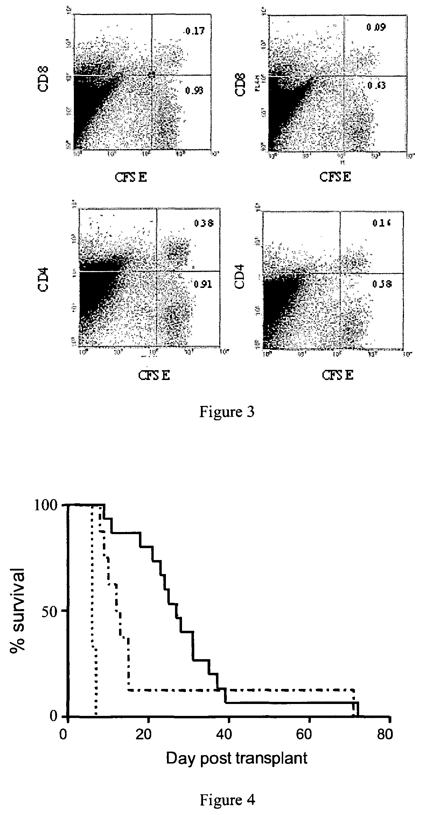

Figure 3: In vitro depleting activity of anti-LAG-3 antibodies. T cells from the spleen were activated for 48h with Con A to induce expression of LAG-3 and labeled with CFSE. 105 cells were injected i.v. to recipients that had been irradiated (4.5 Gy) 3 days before. Twenty-four hours after injection, recipients were sacrificed and the presence of CFSE[+] cells in the CD4[+] and CD8[+] compartments of the spleen analyzed by flow cytometry.

Figure 4: Heart graft survival after anti-LAG-3 antibodies administration. Lew.1A recipients of fully allogeneic (class I and II mismatch) Lew.1W hearts were treated by injections on days 0 and 3 of 200 $\mu$l (dashed line) or 600 $\mu$l (full line) rabbit anti-LAG-3 serum or of 600 $\mu$l pre-immune serum (dotted line). Graft survival was evaluated by daily evaluation of heartbeat. $P < 0.002$ for 600 $\mu$l rabbit anti-LAG-3 serum vs. pre-immune serum.

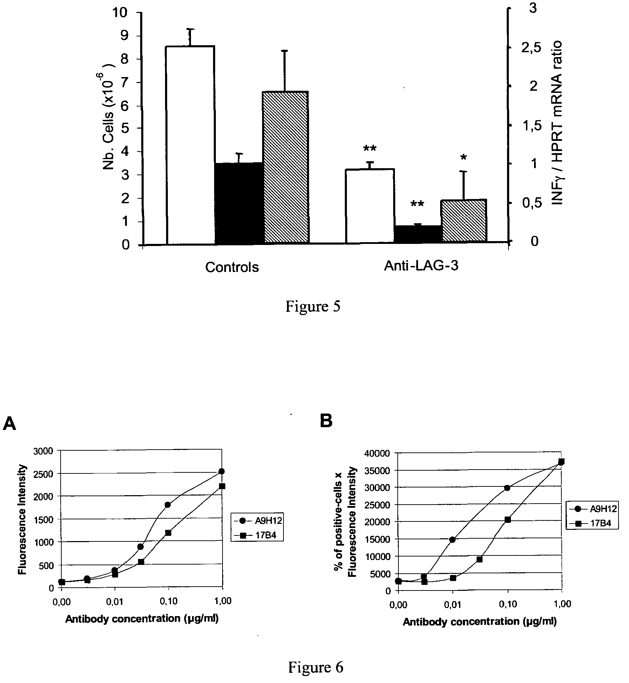

Figure 5: Analysis of Graft infiltrating cells (GICs). GICs were extracted from cardiac allografts on day 5 from control-treated or anti-LAG-3 antibodies treated recipients. Cells were counted and analyzed by flow cytometry for the expression of LAG-3. White bars: total number of GICs. Black bars: LAG-3[+] GICs measured by flow cytometry ($p < 0.01$). Total RNA was also extracted from GICs and messenger for INF$\gamma$ were quantified by qPCR, relative to HPRT expression level (dashed bars; $p < 0.05$).

Figure 6: Comparison of A9H12 binding with the reference LAG-3 specific 17B4 mAb on LAG-3[+] CHO and activated human T cells.

A) hLAG-3-transfected CHO were dissociated from culture plastic using Versene buffer containing cation-chelating agent, incubated with indicated concentrations of A9H12 or 17B4 mAbs for 30 min at 4°C, washed and then incubated with a FITC-conjugated goat anti-mouse IgG+M (H+L) secondary antibody (5 $\mu$g/ml, Coulter) for 30 minutes at 4°C. After washing, cells were analysed by flow cytometry using a FACSCanto (BD Biosciences) and means of fluorescence intensity were plotted as a function of antibody concentration.
B) PBMCs from a healthy volunteer were stimulated for 2 days with SEB (1 $\mu$g/ml, Sigma Aldrich) to induce the expression of LAG-3 on T cells. PBMCs were stained as above. Data represent a weighted percentage, calculated as the percentage of LAG-3[+] cells in PBMCs x mean of fluorescence intensity of the LAG-3[+] cells, plotted as a function of antibody concentration.

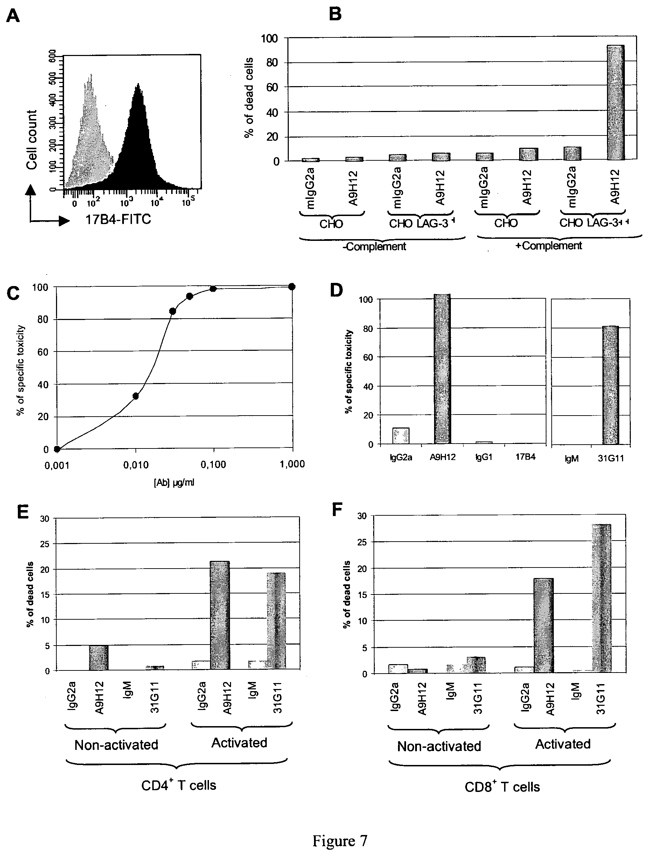

Figure 7: Complement-Dependent Cytotoxicity induced by A9H12 LAG-3 mAb.

A) hLAG-3-transfected and wild type CHO cells were labelled with FITC-conjugated anti-LAG-3 mAb (17B4) and the expression of LAG-3 on cell surface was analysed by flow cytometry using a FACSCanto. The histogram plots represent the mean fluorescence intensity of wt CHO (gray) and LAG-3[+] CHO (dark).
B) hLAG-3-transfected and wt CHO cells were washed in complete medium (MEM supplemented with 10 % heat inactivated Foetal Calf Serum, FCS) and incubated with 0.1 $\mu$g/ml of A9H12 LAG-3 mAb or mIgG2a isotype-control mAb (Southern Biotechnologies) in complete medium for 30 min at 4 °C. Cells were then washed and incubated in complete medium (-Complement) or in MEM supplemented with 10 % of freshly resuspended rabbit serum (Cerdalane Inc.) (+Complement) for 1 hour at 37°C. After washing, cells were stained with 7-AAD (Coulter Inc.) for 15 minutes at room temperature and immediately analysed by flow cytometry to determined the percentage of 7-AAD-positive cells corresponding to dead cells. Data represent the percentage of dead cells in each condition on *hLAG*-3-transfected and wt CHO cells as indicated.
C) LAG-3[+] CHO cells were incubated with indicated concentrations of A9H12 LAG-3 mAb for 30 min at 4°C and then incubated with MEM supplemented with 25 % rabbit serum for 1 hour at 37°C. After washing, cells were stained with 7-AAD (Coulter Inc.) and analysed by flow cytometry. The percentage of specific cytotoxicity is calculated according to the following formula

$$\frac{(\text{Sample Death} - \text{Spontaneous Death}) \times 100}{(\text{Maximal Death} - \text{Spontaneous Death})}$$

where Sample Death is the percentage of 7-AAD-positive cells in each condition, Spontaneous Death, the percentage of 7-AAD-positive cells without mAb and Maximal Death, the percentage of 7-AAD-positive cells with 10 μg/ml mAb.

D) LAG-3[+] CHO cells were incubated with 0.1 μg/ml A9H12, 17B4 or 31G11 LAG-3 mAb or with their corresponding isotype controls (IgG2a, IgGl or IgM, respectively) for 30 min at 4°C and then incubated with MEM supplemented with 25 % rabbit serum for 1 hour at 37°C. Specific cytotoxicity was determined as above with a Maximal Death corresponding to 10 μg/ml A9H12 (left panel) and 0.1 μg/ml A9H12 (right panel).

E) PBMCs were stimulated with SEB (1 μg/ml) to induce LAG-3 expression on T cells and then used as target cells in the CDC assay in the presence of A9H12 or 31G11 LAG-3 mAb or their isotype controls. After staining cells with fluorochrome-conjugated CD3, CD4, CD8, CD25 and 17B4, the percentage of 7-AAD-positive cells was analysed on the indicated T cell subpopulations. Data represent the percentage of dead cells in each population (with spontaneous death in the absence of mAb being subtracted).

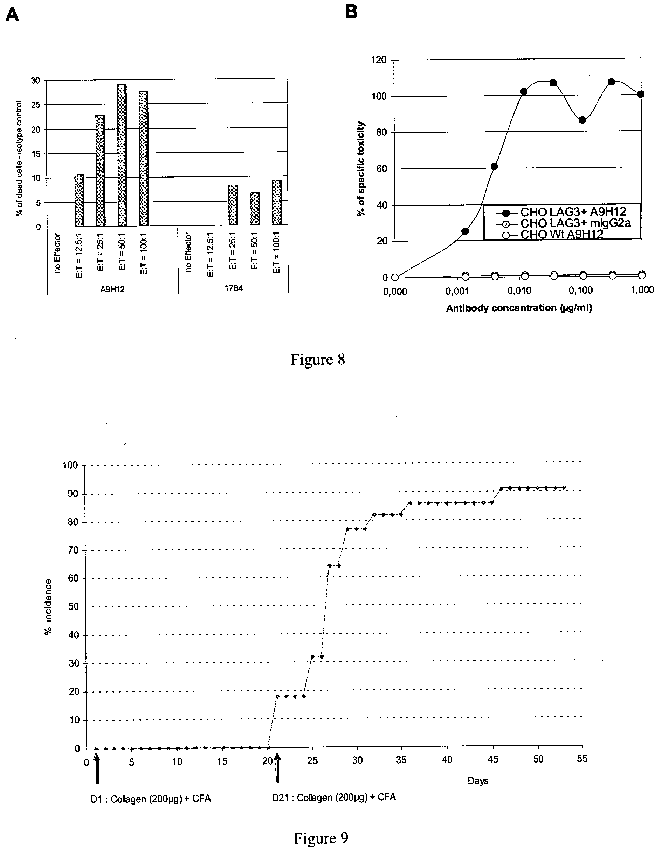

Figure 8: Antibody-Dependent Cell-mediated Cytotoxicity induced by A9H12 LAG-3 mAb

A) Effector cells (PBMCs) were stimulated with IL-2 (100 IU/ml, BD Biosciences) for 1 day. Target cells *(hLAG-3-transfected CHO cells)* were labelled with CFSE (a fluorescent vital dye) and incubated with 1 μg/ml A9H12, mIgG2a, 17B4 or mIgG1 for 20 min at room temperature. Effector cells and target cells were then mixed at indicated E:T ratios (E:T, Effector:Target) and incubated for 16 hours at 37°C. Non-adherent and adherent cells were harvested using Versene reagent, stained with 7-AAD and immediately analysed by flow cytometry to determine the percentage of 7-AAD-positive cells in the CFSE-positive population. Data represent the percentage of dead cells, with the non-specific cell death in the presence of the isotype control being subtracted.

B) CFSE-labelled wild-type or LAG-3[+] CHO target cells were incubated with indicated concentrations of A9H12 or mIgG2a and IL-2-stimulated PBMCs were added at a 50:1 E:T ratio and incubated for 16 hours at 37°C. Cell death was analysed as above and data represent the percentage of dead cells in CFSE-positive cells in the presence of A9H12 or its isotype-matched IgG2a control mAb.

Figure 9: Incidence of arthritis (percentage of mice that developed CIA)
Male DBA/1 mice (n=22) were injected i.d. with bovine type II collagen (200 μg) emulsified in CFA containing 250 μg *M. tuberculosis.*

Figure 10: Construction of the chimeric IMP731 therapeutic antibody.

Figure 11: Expression plasmids for the light (panel A) and heavy (panel B) IMP731 chains.

Figure 12: Final bi-cistronic plasmid construction used for the stable transfection of CHO cells.

Figure 13: IMP731 binding on LAG-3+ CHO and activated human T cells

A) hLAG-3-transfected CHO were dissociated from culture plastic using Versene buffer containing cation-chelating agent, incubated with indicated concentrations of IMP731 Ab or its isotype control hIgG1 (Chemicon) for 30 min at 4°C, washed and then incubated with a FITC-conjugated F(ab)'₂ goat anti-human IgG1 secondary antibody (5 μg/ml, SBA) for 30 minutes at 4°C. After washing, cells were analysed by flow cytometry using a FACSCanto (BD Biosciences) and the means of fluorescence intensity were plotted as a function of antibody concentration.

B) PBMCs from a healthy volunteer were stimulated for 2 days with SEB (1 μg/ml, Sigma Aldrich) to induce the expression of LAG-3 on T cells. PBMCs were stained as above. Data represent a weighted percentage, calculated as the percentage of LAG-3[+] cells in PBMCs x mean of fluorescence intensity of the LAG-3[+] cells, plotted as a function of antibody concentration.

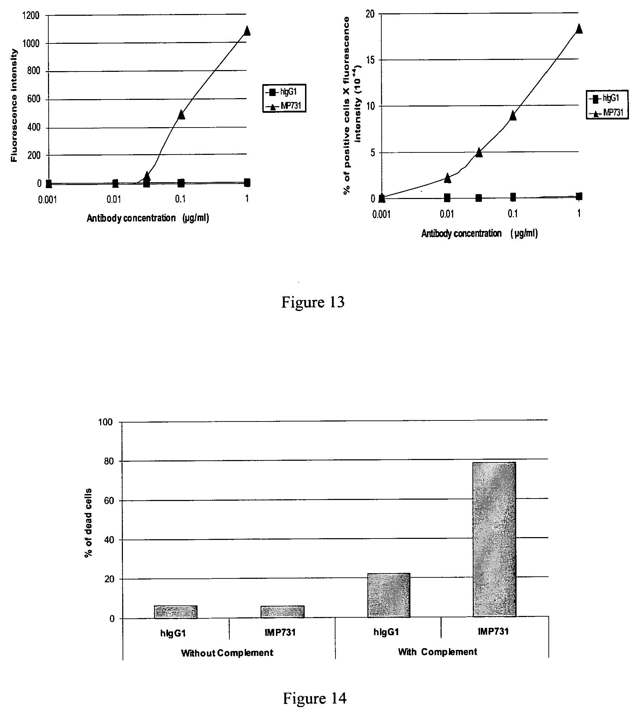

Figure 14: Complement-Dependent Cytotoxicity induced by IMP731 LAG-3 mAb
hLAG-3-transfected CHO cells were incubated with 1 μg/ml of IMP731 Ab or hIgG1 isotype-control mAb (Chemicon) in complete medium (MEM supplemented with 10 % heat inactivated Foetal Calf Serum, FCS) for 30 min at 4 °C. Cells were then washed and incubated in complete medium (without Complement) or in MEM supplemented with 25 % of freshly resuspended rabbit serum (Cerdalane Inc.) (with Complement) for 1 hour at 37°C. After washing, cells were stained with 7-AAD (BD Biosciences) for 15 minutes at room temperature and immediately analysed by flow cytometry to determine the percentage of 7-AAD-positive cells corresponding to dead cells. Data represent the percentage of dead cells in each condition as indicated.

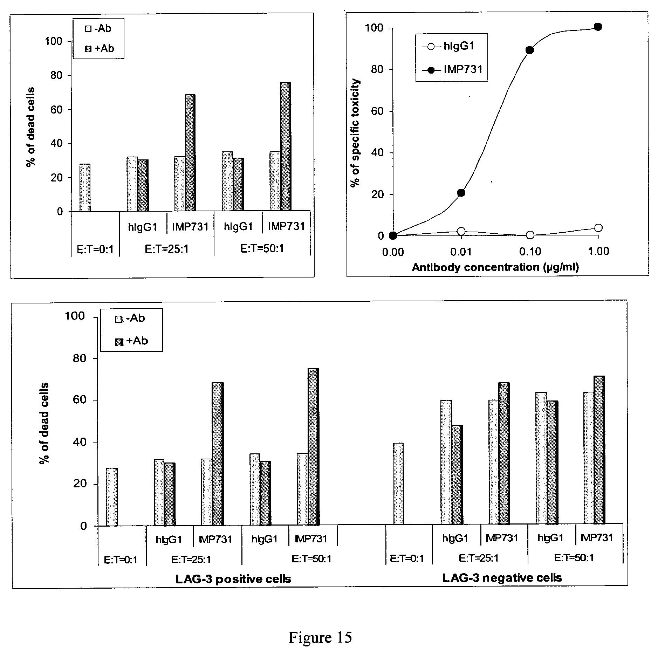

Figure 15: Antibody-Dependent Cell-mediated Cytotoxicity induced by IMP731.

A) Effector cells (PBMCs) were stimulated with IL-2 (100 IU/ml, BD Biosciences) for 1 day. Target cells (*hLAG-3*-transfected CHO cells) were labelled with CFSE (a fluorescent vital dye) and incubated with 1 µg/ml IMP731 or hIgG1 for 10 min at room temperature. Effector cells and target cells were then mixed at indicated E:T ratios (E:T, Effector:Target) and incubated for 16 hours at 37°C. Cells were stained with 7-AAD and immediately analysed by flow cytometry to determine the percentage of 7-AAD-positive cells in the CFSE-positive population. Data represent the percentage of dead cells.

B) CFSE-labelled LAG-3$^+$ CHO target cells were incubated with indicated concentrations of IMP731 or hIgG1 and IL-2-stimulated PBMCs were added at a 50:1 E:T ratio and incubated for 16 hours at 37°C. Cell death was analysed as above in CFSE-positive population. The percentage of specific cytotoxicity, calculated according to the following formula

$$\frac{(\text{Sample Death} - \text{Spontaneous Death})}{(\text{Maximal Death} - \text{Spontaneous Death})} \times 100$$

where Sample Death is the percentage of 7-AAD-positive cells in each condition, Spontaneous Death, the percentage of 7-AAD-positive cells without Ab and Maximal Death, the percentage of 7-AAD-positive cells with 1 µg/ml IMP731 C) Effector cells (PBMCs) were stimulated with IL-2 (100 IU/ml, BD Biosciences) for 1 day. Target cells (hLAG-3$^+$ CHO cells or hLAG-3$^-$ CHO cells) were labelled with CFSE (a fluorescent vital dye) and incubated with 1 µg/ml IMP731 or hIgG1 for 10 min at room temperature. Effector cells and target cells were then mixed at indicated E:T ratios (E:T, Effector:Target) and incubated for 16 hours at 37°C. Cells were stained with 7-AAD and immediately analysed by flow cytometry to determine the percentage of 7-AAD-positive cells in the CFSE-positive population. Data represent the percentage of dead cells.

## V - Detailed description

In a first embodiment, the present invention provides a cytotoxic anti-LAG-3 monoclonal antibody or fragment thereof capable of causing depletion of LAG-3$^+$ activated T cells, wherein said antibody is produced by the hybridoma deposited at the CNCM on April 27, 2007 under the accession number CNCM I-3755, and further wherein said antibody or fragment thereof exhibits complement-dependent cytotoxicity (CDC) and/or antibody-dependent cell cytotoxicity (ADCC).

In a second embodiment, the present invention provides a cytotoxic anti-LAG-3 monoclonal antibody or fragment thereof capable of causing depletion of LAG-3$^+$ activated T cells, wherein said antibody is produced by the hybridoma deposited at the CNCM on April 27, 2007 under the accession number CNCM I-3756, and further wherein said antibody or fragment thereof exhibits complement-dependent cytotoxicity (CDC) and/or antibody-dependent cell cytotoxicity (ADCC).

Antibodies and fragments thereof according to the present invention bind to LAG-3 protein and cause depletion of LAG-3$^+$ activated T cells. Said depletion can be measured by changes in peripheral blood lymphocyte numbers, a tissue or an organ.

The present invention relates preferably to human LAG-3 protein (hLAG-3 also named hereafter LAG-3).

Lymphocyte activation gene-3 (LAG-3, CD223) is up-regulated during the early stages of T-cell activation. The present invention is based on the analysis of the effects of cytotoxic antibodies against LAG-3 in acute cardiac allograft rejection and in rheumatoid arthritis (*in vivo* animal studies) and in *in vitro* experiments where selected LAG-3 monoclonal antibodies are efficient at low doses (<0.1 µg/ml) at depleting LAG-3$^+$ activated effector T cells.

Fully vascularized heterotopic allogeneic heart transplantation was performed in rats across a full-MHC mismatch barrier (LEW.1W into LEW.1A). Recipients received two injections (day 0 and 3) of antibodies directed to the extraloop domain of LAG-3 or control antibodies. Graft survival, histology, mRNA transcripts and alloreactivity of lymphocytes were tested.

It was first noted that LAG-3 mRNA molecules accumulate in cardiac allografts undergoing rejection, but not in peripheral lymphoid organs. Administration of anti-LAG-3 antibodies inhibited graft infiltration by effectors mononuclear cells and prolonged allograft survival from 6 days in control antibodies-treated recipients to a median of 27 days.

It was found that cells expressing LAG-3 infiltrate rejected heart allografts and that targeting LAG-3 using cytotoxic antibodies as induction monotherapy delays acute rejection by reducing graft infiltration by T cells and monocytes.

Experiments showing that short courses of CD40L antibody therapy could achieve long-term graft survival in mice and primates have been initially interpreted as an effect of costimulation blockade. However, Monk et *al*. (2) showed that much of the efficacy of anti-CD40L therapy derives not from costimulation blockade, but from destruction of activated T cells. The outcome is a selective purging of potentially aggressive T cells that have experienced antigen.

Collagen-induced arthritis (CIA) is a well-described animal model for rheumatoid arthritis. Collagen-induced arthritis is an autoimmune disease inducible in rats, mice and primates by immunization with heterologous type II collagen. The resulting joint pathology resembles rheumatoid arthritis with synovial proliferation, cell infiltration, cartilage erosion and bone resorption in the most severe cases (12).

Using particular immunization protocols, early studies have established a hierarchy of responsiveness to CIA linked to the H-2 haplotype, with H-2$^q$ (e.g. DBA/1 mice) being the most and H-2$^b$ (e.g.C57BL/6 mice) amongst the least responsive strains. However, some studies have shown that responsiveness to CIA may be less restricted by the MHC class II than previously thought and may be just as dependent on immunization conditions (13). The variety of type II collagen (CII) from different species and the preparation of complete Freund's adjuvant (CFA) with different concentrations of *Mycobacterium tuberculosis* were important parameters for arthritis development. Inglis et al. have shown that chicken, but not bovine, CII was capable of inducing disease in C57BL/6 mice, with an incidence of 50% to 75%. This is in contrast to DBA/1 mice, in which bovine, mouse and chicken CII all induced disease, with an incidence of 80% to 100%. The phenotype of arthritis was milder in C57BL/6 mice than in DBA/1 mice, with less swelling and a more gradual increase in clinical score (14). Moreover, male mice are frequently preferred for CIA studies, as the incidence of arthritis is somewhat higher in male than in female mice.

In mice, CIA is induced by an i.d. injection of type II collagen (CII) in the presence of CFA, usually followed by an i.p. boost injection of CII, without adjuvant, 21 days later. However, there are reported variations for almost every aspect of the immunization procedure and even in the highly susceptible DBA/1 strain the time of onset, severity and incidence of CIA can be variable (13, 15).

Therapeutic antibodies for the treatment of auto-immune diseases have already been described, like the TNFα mAbs in rheumatoid arthritis. By definition, LAG-3 (Lymphocyte Activation Gene-3) is a marker for recently activated effector T cells. Depleting these effector LAG-3+ T cells will lead to targeted immunosuppression (i.e. only activated T cells are suppressed, not all T cells as with corticoids or cyclosporin). This very specific immunosuppression should lead to higher therapeutic indices compared to classical immunosuppressive agents or to therapeutic antibodies (e.g. Humira, Remicade) or soluble receptors (e.g. Enbrel) blocking TNFα. Thus, LAG-3 is a promising target available for a therapeutic depleting mAb approach to eliminate auto-reactive activated effector T cells.

Molecules disclosed herein that bind to LAG-3 protein and cause depletion of LAG-3+ activated T cells include antibodies (mono or polyclonal, preferably monoclonal) and fragments thereof, peptides and organic small molecules.

Disclosed herein is a cytotoxic anti-LAG-3 monoclonal antibody or fragment thereof that causes depletion of more than 30% preferably more than 50 % of LAG-3+ activated T cells.

Cytotoxic anti-LAG-3 monoclonal antibody or fragment thereof disclosed herein comprise antibodies with a murine IgG2a or a human IgG1 Fc region giving strong CDC or ADCC properties.

Disclosed herein is a cytotoxic anti-LAG-3 monoclonal antibody or fragment thereof, which exhibits (i) more than 50 % of maximal CDC activity at a mAb concentration of less than 0.1 μg/ml and/or (ii) more than 50 % of maximal ADCC activity at a mAb concentration of less than 0.1 μg/ml.

Cytotoxic anti-LAG-3 monoclonal antibodies of the present invention, causing depletion of LAG-3+ activated T cells, can be produced by methods well known to those skilled in the art.

Antibodies generated against CD223 polypeptides can be obtained by administering, in particular by direct injection, CD223 polypeptides to an animal, preferably a non-human. The antibody so obtained will then bind the CD223 polypeptides itself. In this manner, even a sequence encoding only a fragment of the CD223 polypeptide can be used to generate antibodies binding the whole native CD223 polypeptide.

For preparation of monoclonal antibodies, any technique which provides antibodies produced by continuous cell line cultures can be used. Examples include the hybridoma technique (9), the trioma technique, the human B-cell hybridoma technique (10).

Techniques described for the production of single chain antibodies (US. Pat. No. 4,946,778) can be readily used to produce single chain antibodies to CD223 polypeptides. Also, transgenic mice may be used to express humanized antibodies to immunogenic CD223 polypeptides.

A first monoclonal antibody according to the present invention, called A9H12, is produced by the hybridoma deposited at the CNCM on April 27, 2007 under the access number CNCM I-3755.

A second monoclonal antibody according to the present invention, called 31G11, is produced by the hybridoma deposited at the CNCM on April 27, 2007 under the access number CNCM I-3756.

In one embodiment, there is provided a cytotoxic anti-LAG-3 monoclonal antibody or fragment thereof according to the present invention for use in treating or preventing organ transplant rejection or for use in treating a T-cell mediated autoimmune disease.

Disclosed herein is the use of a cytotoxic anti-LAG-3 monoclonal antibody or fragment thereof for the manufacture of a medicament for treating or preventing organ transplant rejection or for treating autoimmune disease.

Disclosed herein is a method for treating or preventing organ transplant rejection or for treating autoimmune disease. Said method comprises the administration to a mammalian subject a therapeutically effective amount of a cytotoxic anti-LAG-3 monoclonal antibody or fragment thereof.

Organ transplant rejection refers to the graft of an organ in an allogenic host. It may be useful for treating organisms suffering from conditions resulting in an abnormally high T-cell population or deleterious T-cell activity, for example graft rejection mediated by host T-cells, graft vs. host disease and T-cell mediated autoimmune and inflammatory diseases such as rheumatoid arthritis, type 1 diabetes, muscular sclerosis, etc. The methods of the invention may be applied to any organism which contains T-cells that express CD223. This includes, but is not limited to, any mammal and particularly includes humans and mice.

Auto-immune deseases are diseases in which the subject's own immune system reacts against the subject cells. Auto-immune disease which are amenable to treatments according to the present invention include autoimmune hemolytic anemia, autoimmune thrombocytopenia purpura, Goodpasture's syndrome, pemphigus vulgaris, acute rheumatic fever, mixed essential cryoglobulinemia, systemic lupus erythematosus, insulin-dependent diabetes mellitus, rheumatoid arthritis, Grave's disease, Hashimoto's thyroiditis, myasthenia gravis, and multiple sclerosis.

Also disclosed herein is a method for depleting LAG-3+ activated T cells from a sample from a patient, comprising reacting the sample with an antibody composition comprising an antibody described above.

A pharmaceutical composition according to the present invention comprises from 30 to 300 mg per dose of a cytotoxic monoclonal antibody of the present invention and one or more pharmaceutically acceptable carriers and/or diluents for administration to a mammal. The pharmaceutical composition of the present invention may be specially formulated for administration in solid or liquid form.

EXAMPLE 1 : LAG-3-positive cells targeted with cytotoxic antibodies

Material and methods

*Animals and transplantations*

Eight- to 12-week-old male Lewis.1W (LEW.1W, haplotype RT1$^u$) and Lewis.1A (LEW. 1A, haplotype RT1$^a$) congeneic rats (Centre d'Elevage Janvier, Le Genest-Saint-Isle, France), differed in their entire MHC region. Heterotopic LEW.1W heart transplantation was performed as previously described (11). Graft survival was evaluated by palpation through the abdominal wall.

*Anti-LAG-3 antibodies*

A synthetic peptide corresponding to the extraloop domain of the rat LAG-3 protein (NCBI accession nb DQ438937; peptide DQPASIPALDLLQGMPSTRRHPPHR) was linked to ovalbumin and used to immunise two rabbits. Pre-immune and immune sera, collected on day 63 after the 4$^{th}$ immunisation, were assayed by ELISA on immunogen and peptide and by flow cytometry on Con-A activated rat spleen cells. Pre-immune sera were negative in both assays. Pooled immune sera presented a titer (dilution for 50% signal) of 1/60000 by ELISA and of 1/1000 by FACS, and presented a specificity for activated T cells.

*Complement-dependant cytotoxicity assay*

Complement-mediated antibody-dependent cytotoxicity was tested using rabbit sera against Lewis 1A T cells in a $^{51}$Cr release assay. A total of $2 \times 10^6$ Lewis 1A T cells were labelled with 30 $\mu$Ci of $^{51}$Cr for 90 min at 37°C in RPMI (GIBCO) with 10% FCS. After three washes, T cells were distributed in 96 V-bottomed plates and incubated with rabbit complement and serial dilutions of heat-inactivated rabbit serum. After 4 h at 37°C, $^{51}$Cr release was measured in the supernatants using a scintillation counter. Specific cytotoxicity was calculated according to the following formula: (experimental release - spontaneous release) $\times$ 100/(maximum release - spontaneous release).

*In vivo antibody-induced cytotoxicity*

Cytotoxic activity of anti-LAG-3 antibodies against LAG-3$^+$ cells was evaluated in vivo. ConA-activated (48h) LEW.1W splenocytes were labelled with the CFSE and transferred (10$^8$ cells) into irradiated (4.5 Gy, day -3) LEW.1A recipients, together with anti-LAG-3 antibodies. On day 1, recipients were sacrificed and the presence of CFSE-positive cells evaluated by flow cytometry in lymphoid organs and in the blood.

*Immunostaining*

Graft samples were embedded in Tissue Tek (OCT Compound, Torrance, CA, USA), snap-frozen in liquid nitrogen, cut into 5 μm sections and fixed in acetone. Endogenous biotin activity was blocked using the Dako biotin blocking system (Dako, Trappes, France). Sections were then labelled by a three-step indirect immunoperoxidase revelation. The area of each immunoperoxidase-labeled tissue section infiltrated by cells was determined by quantitative morphometric analysis. Positively stained cells on each slide were counted by morphometric analysis using point counting analysis (14) with a 121-intersection squared grid in the eyepiece of the microscope. Briefly, the percentage of the area of each graft section occupied by cells of a particular antigenic specificity (area infiltrate) was calculated as follows: [number of positive cells under grid intersection/(total number of grid intersections = 121)] x 100. The graft sections were examined at a magnification of x400. The accuracy of the technique is proportional to the number of points counted. Thus, to maintain a SE of <10%, 15 fields were counted for each labeled section. Results are expressed as the percentage of the area of the tissue section infiltrated by leukocytes (determined with OX1, OX30 labeling) and the phenotypic composition of the infiltrate and subpopulations which are related to the percentage of total leukocytes and are expressed as the percentage of leukocytes.

*Graft_infiltrating cell extraction staining*

Dilacerated hearts were digested with collagenase D (2 mg/ml; Boehringer Mannheim) for 10 min at 37°C. Cells were then collected by extraction through a stainless steel mesh. The resulting suspension was then clarified by Ficoll isolation.

*Quantitative RT-PCR*

Real-time quantitative PCR was performed in an Applied Biosystems GenAmp 7700 Sequence Detection System using SYBR Green PCR Core Reagents (Applied Biosystems, Foster City, CA). The following oligonucleotides were used in this study: rat LAG-3: upper primer is 5'-ATATGAATTCACAGAGGAGATGAGGCAG-3' and lower primer is 5'-ATATGAATTCTCCTGGTCAGAGCTGCCT-3'. Rat INF-g: upper primer is 5'-TGGATGCTATGGAAGGAAAGA-3' and lower primer is 5'-GATTCTGGTGACAGCTGGTG-3'. Rat HPRT: upper primer is 5'-CCTTGGTCAAGCAGTACAGCC-3' and lower primer is 5'-TTCGCTGATGACACAAACATGA-3'. A constant amount of cDNA corresponding to the reverse transcription of 100 ng of total RNA was amplified in 25 μl of PCR mix containing 300 nM of each primer; 200 μM dATP, dGTP, dCTP; 400 μM dUTP; 3 mM MgCl$_2$; 0.25 U of uracyl-N-glycosylase; 0.625 U of AmpliTaq Gold DNA polymerase. The mix was subjected to 40 cycles of amplification. The real-time PCR data were plotted as the $\Delta R_n$ fluorescence signal vs. the cycle number. The $\Delta R_n$ values were calculated by the Applied Biostystems 7700 sequence detection software using the formula: $\Delta R_n = (R_n^+) - (R_n^-)$, where $R_n^+$ is the fluorescence signal of the product at any given time, $R_n^-$ is the mean fluorescence signal during cycles 3-13 and referred to as the baseline. The C$_t$ value is defined as the cycle number at which the $\Delta R_n$ crosses a threshold. The threshold is set above the background fluorescence to intersect the exponential portion of the amplification curve of a positive reaction. The Ct is inversely proportional to the log amount of template within the PCR.

*Statistical analyses*

Statistical significance was evaluated using aa Mann-Whitney test for the comparison of two groups. Graft survival was evaluated by Kaplan-Meier analysis using the log rank test.

Results

*LAG-3 mRNA expression in rejected allograft and lymphoid organs*

LAG-3 is expressed by activated T cells in inflamed lymphoid organs and tissues (7). In order to see if LAG-3 is also expressed in rejected allografts, hearts grafts from LEW.1W to LEW.1A rat recipients were analyzed on day 5 (rejection occurring on day 6). Messenger RNA for LAG-3 was analyzed and compared with allografts receiving a tolerance-inducing regiment (anti-CD28 antibodies + CSA, as described (16)) and with isografts. Rejected allografts presented a 7-fold and a 25-fold accumulation of LAG-3 mRNA as compared with tolerated and syngeneic grafts, respectively (Figure 1A). Such an accumulation was not detected in lymph nodes (Figure 1B) or in the spleen of rejecting recipients (Figure 1C).

*Mechanism of action of anti-LAG-3 antibodies*

Anti-LAG-3 antibodies were produced in rabbits by immunization with a synthetic peptide from the extra-loop of LAG-3 Ig-like N-terminal domain , involved in the interaction of LAG-3 with Class II (ref PNAS Huard 1997). Post-immune serum, as well as the IgG fraction, stained <1% of rat spleen cells and 40% of rat spleen cells activated for 48h with ConA, PMA + ionomycin or PHA. Pre-immune serum was negative (data not shown). In order to characterize the effect of anti-LAG-3 antibodies on LAG-3$^+$ cells, complement and ADCC-dependant cytotoxicity was assayed in vitro. Fifteen % of ConA-activated spleen cells were lysed in the complement-dependant cytotoxicity assay (Figure 2). Given that only 40% of the ConA-activated target cells expressed LAG-3, this assay revealed that about 37 % of the LAG-3$^+$ spleen cells present in the preparation were lysed in vitro as a result of complement activation.

*In vivo,* the depleting activity of anti-LAG-3 antibodies was estimated by measuring the fate of CFSE-labeled activated T cells adoptively transferred to irradiated rat recipients. One day after the injection of therapeutic doses of anti-LAG-3 immune serum, only half the amount of CFSE$^+$/CD4$^+$ and CFSE$^+$/CD8$^+$ cells could be recovered from the spleen, as compared with similar injections of pre-immune serum (Figure 3).

*Anti-LAG-3 antibodies delay heart allograft rejection*

From preliminary pharmacokinetic observations, we established that two i.v. injections of 600 $\mu$l of anti-LAG-3 rabbit serum on days 0 and 3 resulted in the maintenance of anti-LAG-3 binding activity in recipient's serum for at least 2 weeks. This treatment delayed cardiac allograft rejection from 6 days in untreated and control-treated recipients to a median of 27 days. All recipients, however, eventually rejected their graft within 10 weeks (Figure 4). On day 5, grafts from control-treated recipients were heavily infiltrated by activated T cells and this infiltrate was less important in anti-LAG-3 treated recipients. Infiltration by CD25$^+$ cells and NK cells, however, was not modified by the treatment. Since our anti-LAG-3 antibodies do not recognize LAG-3 in immunohistology, LAG-3 expression by graft infiltrating cells (GICs) was analyzed by flow cytometry after extraction. An average of 8.5 10$^6$ $\pm$ 0.76 GICs could be recovered from control rejected grafts. From heart allografts from anti-LAG-3 treated recipients, only 3.16 $\pm$0.44 10$^6$ GICs could be recovered (n=3; p< 0.005). GICs contained 41.17 $\pm$ 1 % LAG-3$^+$ cells in controls (i.e. 3.5 10$^6$ cells) versus 22.2 $\pm$ 0.9 % in treated animals (i.e. 0.7 10$^6$ cells; n=3; p<0.0005; Fig. 5). Analysis of mRNA transcript reinforced these observations that infiltration of heart graft by mononuclear cells was reduced since we measured four times less INF$\gamma$ mRNA molecules in treated grafts (Figure 5).

*Anti-LAG-3 antibodies inhibit ongoing acute heart allograft delay rejection*

In order to investigate whether anti-LAG-3 antibodies might serve as a treatment of an ongoing acute allograft rejection, we grafted LEW.1W hearts into LEW.1A allogenic recipients that we maintained untreated over 3 or 4 days. At that time, recipients received an injection of 600 microliter of control or anti-LAG-3 rabbit serum. Control-treated recipients rejected the allografts on day 5 whereas anti-LAG-3 antibodies-treated recipients rejected only on day 11 (Table 1).

Table 1

| Treatment | Day of rejection | Median survival |
|---|---|---|
| Control serum on day 3 | 5, 5, 5 | 5 |
| Control serum on day 4 | 5, 5, 5 | 5 |
| Anti-LAG-3 serum on day 3 | 12, 13, 9 | 12 (p<0.05 vs. control) |
| Anti-LAG-3 serum on day 4 | 10, 13, 13, 19 | 12.5 (p<0.05 vs. control) |

Table 1: Heart graft recipients were treated on day 3 or 4 with control or anti-LAG-3 antibodies. Rejection was monitored by daily heart palpation.

EXAMPLE 2: Generation of new high affinity hLAG-3 mAbs

Material and methods

Mice were immunized 3 times with hLAG-3-transfected CHO cells ($10^7$ cells, intra-peritoneal injection), followed by a boost *i.v.* injection with 10 $\mu$g IMP321, the clinical-grade hLAG-3Ig recombinant protein. Three days after the boost, splenocytes were fused with the X63.AG8653 fusion partner to yield hybridoma cells. Supernatants from hybridomas were screened for their specific binding (FACS analysis) on hLAG-3-tranfected CHO versus wild type (wt) CHO cells.

One murine IgG2a antibody (580.1E9H3A9H12, called A9H12) was selected, subcloned to yield a stable cell line and further characterized for its potency to deplete LAG-3$^+$ cells through CDC (complement-dependent cytotoxicity) and ADCC (antibody-dependent cell-mediated cytotoxicity), given that the murine IgG2a Fc region is known to be the most efficient Fc isotype in mice at delivering these activities, even on heterologous cells (i.e. CHO cells or human PBMCs). Similarly, a second IgM antibody (31G11E8, called 31G11) was also selected.

Results

Dose-dependent binding of A9H12 was first compared to the reference LAG-3-specific 17B4 mAb on hLAG-3-tranfected CHO cells and on LAG-3$^+$ *in vitro* activated human T cells (Figure 6). A9H12 displayed a greater binding than the reference 17B4 mAb on both cell types. For instance, significant binding of A9H12 to activated human T cells was observed with a concentration as low as 0.01 $\mu$g/ml.

For CDC testing, the target cells used in this assay were LAG-3$^+$ CHO cells compared to wt CHO cells (Figure 7A). Both types of cells were incubated for 1 hour at 37°C with either A9H12, its murine isotype-matched IgG2a negative control mAb, 31G11, its murine isotype-matched IgM negative control or the reference 17B4 (IgG1) mAb and rabbit serum containing active complement. Cell viability was then assessed using 7-Amino-Actinomycin D (7-AAD), a fluorescent dye labelling cells which have lost their membranous integrity, a phenomenon which appeared rapidly after death. The percentage of 7-AAD-positive CHO cells (i.e. dead target cells) was determined by flow cytometry analysis. A9H12 displayed a potent and specific cytotoxic activity in this CDC assay, killing only LAG-3$^+$ CHO cells in the presence of complement (Figure 7B). The anti-LAG-3 Ab was titered down to determined the efficacy of the antibody to activate CDC at low concentration of antibody. A9H12 efficiently induced LAG-3$^+$ CHO cells killing at a concentration as low as 0.01 $\mu$g/ml (Figure 7C). The IgG1 17B4 antibody was also tested in this assay and had no effect (Figure 7D, left panel), showing that not all LAG-3 mAbs could induce cytotoxicity in this bioassay. As observed with A9H12, the second 31G11 LAG-3-specific mAb also induced LAG-3$^+$ CHO cells killing (Figure 7D, right panel).

The CDC bioassay was also performed on PBMCs stimulated with the superantigen SEB. The cytotoxicity of A9H12 and 31G11 were analysed on both activated (namely CD25$^+$/LAG-3$^+$ cells) and non-activated (namely CD25$^-$/LAG-3$^-$ cells) CD4$^+$ helper T and CD8$^+$ cytotoxic T cells. Only activated CD4$^+$ and CD8$^+$ T cells were specifically killed by A9H12 and 31G11 (Figure 7E), showing that activated human T cells are susceptible to A9H12- or 31G11-specific killing in the presence of complement.

For ADCC testing, PMBCs were stimulated for one day with IL-2 to serve as effector cells and LAG-3$^+$ CHO cells were labelled with the vital dye CFSE to serve as target cells. In the presence of A9H12, PBMCs were able to kill a significant percentage of LAG-3$^+$ CHO cells and this effect was increased with the number of effector cells (Figure 8A). In the presence of 17B4, only a small percentage of target cells was killed even at a high E:T ratio (Figure 8A), showing that not all LAG-3 mAbs could induce cytotoxicity in this bioassay. The A9H12 LAG-3 mAb was titered down to determine the efficacy of the antibody to induce ADCC at low concentration of antibody. A9H12 efficiently induced LAG-3$^+$ CHO cells killing at a concentration as low as 0.01 $\mu$g/ml (Figure 8B).

EXAMPLE 3: Testing depleting LAG-3 antibodies in a collagen-induced arthritis mouse model

Material and methods

*Animals and materials*

Male DBA/1 (H-2$^q$) mice, 8-10 weeks old, were obtained from Janvier Laboratories. All animal experiments were performed according to local guidelines. BovineCII (joint cartilage) was purchased from BioCol. Incomplete Freund's adjuvant was provided by Sigma. Heat-killed M. *tuberculosis* H37Ra was purchased from Difco Laboratories.

*Induction of collagen-induced arthritis (CIA)*

The induction and assessment of CIA were performed as previously described in two publications (13, 15).

Complete freund's adjuvant was prepared by mixing 100 mg heat-killed *Mycobacterium tuberculosis* in 13.3 ml IFA (final concentration 7.5 mg/ml). Bovine CII was dissolved at 3 mg/ml in 10 mM acetic acid overnight at 4°C. An emulsion was formed by mixing 2 volumes of CII with 1 volume of CFA. The CII solution and the emulsion with CFA were always freshly prepared. Male DBA/1 mice were intra-dermally injected at the base of the tail with a total of 100 $\mu$l of emulsion containing 200 $\mu$g CII and 250 $\mu$g M. *tuberculosis* on day 1 (D1). The injection was repeated at day 21 (D21). As control, three mice were injected with the emulsion of CFA without CII.

*Clinical assessment of arthritis*

Mice were examined for signs of arthritis three times a week from day 22. The disease severity was determined with the following scoring system for each limb: score 0 = normal; score 1 = swelling of footpad or joint; score 2 = swelling of footpad and 1 or 2 joints; score 3 = swelling of footpad and 3 or 4 joints; score 4 = swelling of footpad and all joints. Each paw was graded, and the 4 scores were summed so that the maximum possible score was 16 per mouse. Incidence was expressed as the percentage of mice with an arthritis score $\geq$ 1.

Results

CIA was induced by i.d. injections of bovine type II collagen (CII) emulsified in CFA containing 250 $\mu$g M. *tuberculosis.* After one injection, 4 out of 22 mice had developed arthritis at D21. Two weeks after the second injection, at D35, 80-90% of the mice had developed clinical signs of arthritis (Figure 9). The mice exhibited clinical scores covering the full range of responses from 1 to 16 with some limbs showing severe swelling of the footpad, ankle/wrist joint and digits (Table 2). None of the control animals (injected with CFA without CII) developed signs of arthritis (data not shown).

Table 2

| Days | Mean | SEM |
|------|------|-----|
| 25 | 2.2 | 0.9 |
| 27 | 2.7 | 0.8 |
| 29 | 5.7 | 1.0 |
| 32 | 9.2 | 1.5 |
| 34 | 10.5 | 1.5 |
| 36 | 10.9 | 1.6 |
| 39 | 10.8 | 1.7 |
| 41 | 10.9 | 1.6 |
| 43 | 11.2 | 1.5 |
| 46 | 11.7 | 1.3 |
| 53 | 13.1 | 1.2 |
| 55 | 13.3 | 1.1 |

Table 2: Mean clinical scores ($\pm$SEM) over 55 days. Male DBA/1 mice (n=10) were injected i.d. with bovine type II collagen (200 mg) emulsified in CFA containing 250 mg M. tuberculosis at D1 and D21.

Our results show that with the CIA protocol used, it is possible to obtain a high percentage (80-90%) of mice developing signs of arthritis. This experimental protocol provides a model to evaluate the therapeutic effect of depleting LAG-3 antibodies (specific for mouse LAG-3) in auto-immune diseases.

200 $\mu$g of the depleting LAG-3 mAb (A9H12 or 31G11) are injected i.p. or i.v. on day 15 and 25. Both a significant decrease in arthritis incidence and a significant lowering of mean clinical scores are involved.

EXAMPLE 4: Complement-dependent cytotoxicity (CDC) and antibody-dependent cell-mediated cytotoxicity (ADCC) induced by IMP731

Materials and methods

A new murine mAb with depleting properties, A9H12, has been shown to recognize also baboon and macaque monkey LAG-3$^+$ cells with high avidity and had been chosen as our lead depleting therapeutic mAb (*Immu Tune* IMP731).

A9H12 has been chimerized with a human IgG1 Fc region using standard genetic engineering and PCR protocols, to give CDC (complement-dependent cytotoxicity) and ADCC (antibody-dependent cell cytotoxicity) properties.

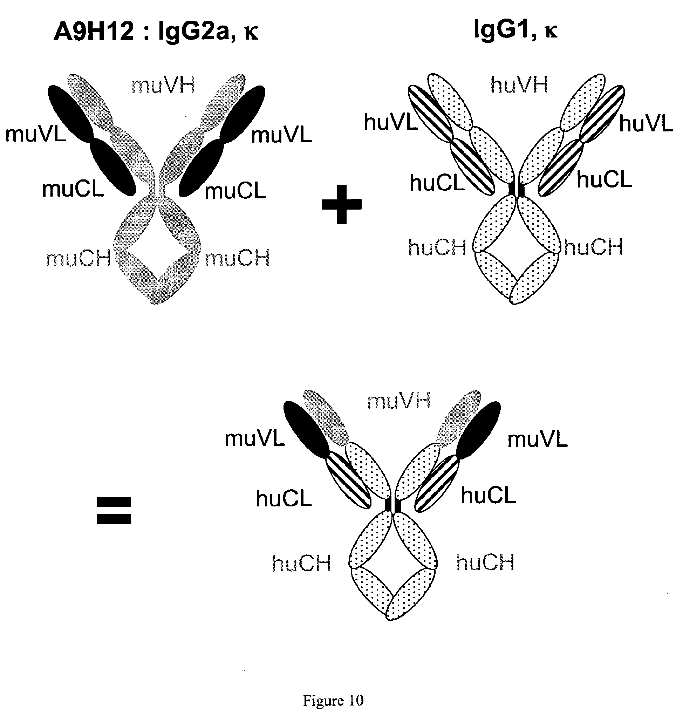

The A9H12 VH and VL cDNA sequences derived from A9H12 hybridoma cell mRNA were fused upstream of the human CH1-hinge-CH2-CH3 IgG1 domains and Ckappa chains, respectively (Figure 10).

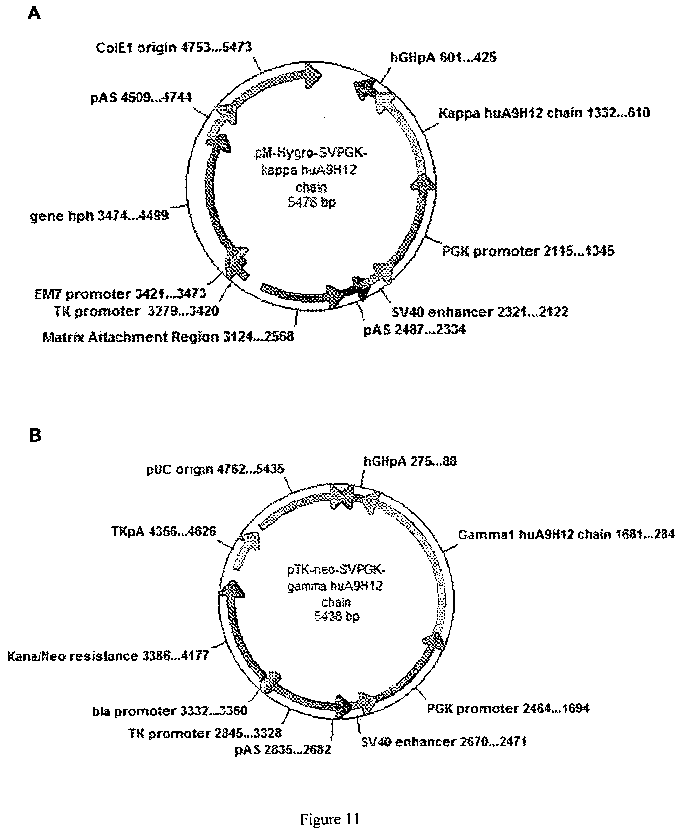

The two light and heavy IMP731 chimeric chains were independently cloned into separate expression plasmids (Figure 11 panel A and B, respectively) under the control of the PGK (or SRalpha in another construction, not shown) promoter. These 2 plasmids were cotransfected (transitory transfection) together into CHO cells and IMP731 was purified from the culture supernatant at day 2 or 3 by using protein A column affinity capture and elution at pH 3. After neutralisation with Tris-HCl the purified IMP731 antibody was tested in CDC and ADCC experiments for its ability to kill LAG-3+ target cells.

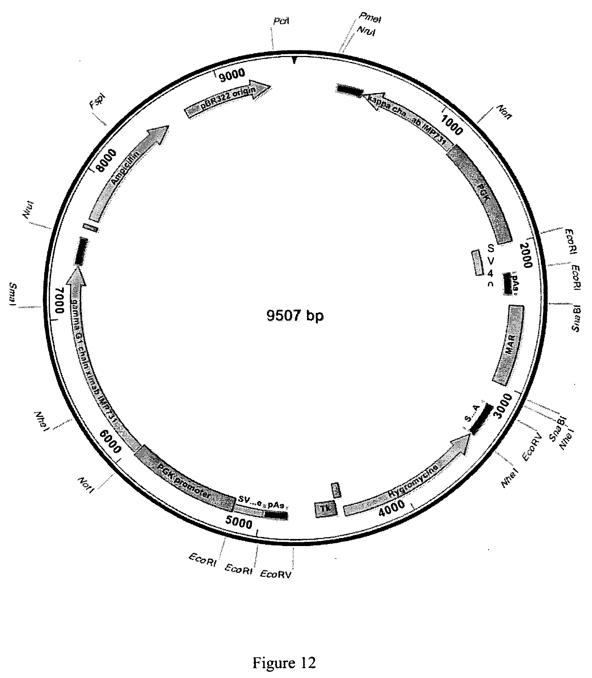

The two IMP731 light and heavy chains were then cloned together with the PGK (or SRalpha, not shown) promoter in a head-to-tail situation for coordinated expression of the two IMP731 chains from the same integration site (Figure 12). This bi-cistronic IMP731 expression plasmid was used for stable transfection and selection of high-productivity (e.g. more than 20 picogramme IMP731 protein per million CHO-S cells per day) CHO-S cells using increasing concentrations of hygromycine in serum-free medium.

Results

Dose-dependent binding of IMP731 was first assessed on hLAG-3-tranfected CHO cells (Figure 13A) and on LAG-3+ in vitro activated human T cells (Figure 13B). IMP731 displayed a significant binding on both cell types with a concentration as low as 0.01 μg/ml for activated T cells.

For complement dependent cytotoxicity (CDC) testing, the target cells used in this assay were LAG-3+ CHO cells (Figure 14). Cells were incubated either with IMP731 or its human isotype-matched IgG1 negative control and then with rabbit serum containing active complement for 1 hour at 37°C. Cell viability was then assessed using 7-Amino-Actinomycin D (7-AAD). 7-AAD is a fluorescent dye labelling cells which have lost their membranous integrity, a phenomenon which appeared rapidly after death. The percentage of 7-AAD-positive CHO cells (i.e. dead target cells) was determined by flow cytometry analysis. IMP731 displayed a potent and specific cytotoxic activity in this CDC assay, killing only LAG-3+ CHO cells in the presence of complement (Figure 14).

For antibody-dependent cell-mediated cytotoxicity (ADCC) testing, PBMCs were stimulated for one day with IL-2 to serve as effector cells and LAG-3+ CHO cells were labelled with the vital dye CFSE to serve as target cells. In the presence of IMP731, PBMCs were able to kill a high percentage of LAG-3+ CHO cells (Figure 15A). IMP731 LAG-3 Ab was tittered down to determine the efficacy of the antibody to induce ADCC at low concentration of antibody. IMP731 significantly induced LAG-3+ CHO cells killing at a concentration as low as 0.01 μg/ml (Figure 15B). LAG-3+ but not LAG-3- cells were killed by the addition of IMP731 in this assay (Figure 15C).

It appeared that binding and functional activities of IMP371 were similar to the parental A9H12 murine mAb produced by hybridoma cells.

References

1. Waldmann H. The new immunosuppression: just kill the T cell. Nat Med 2003; 9 (10): 1259.

2. Monk NJ, Hargreaves RE, Marsh JE, et al. Fc-dependent depletion of activated T cells occurs through CD40L-specific antibody rather than costimulation blockade. Nat Med 2003; 9 (10): 1275.

3. Andre P, Prasad KS, Denis CV, et al. CD40L stabilizes arterial thrombi by a beta3 integrin--dependent mechanism. Nat Med 2002; 8 (3): 247.

4. Avice MN, Sarfati M, Triebel F, Delespesse G, Demeure CE. Lymphocyte activation gene-3, a MHC class II ligand expressed on activated T cells, stimulates TNF-alpha and IL-12 production by monocytes and dendritic cells. J. Immunol. 1999; 162: 2748.

5. Andreae S, Piras F, Burdin N, Triebel F. Maturation and activation of dendritic cells induced by lymphocyte activation gene-3 (CD223). J Immunol 2002; 168 (8) : 3874.

6. Andreae S, Buisson S, Triebel F. MHC class II signal transduction in human dendritic cells induced by a natural ligand, the LAG-3 protein (CD223). Blood 2003; 102 (6): 2130.

7. Triebel F. LAG-3: a regulator of T-cell and DC responses and its use in therapeutic vaccination. Trends Immunol 2003; 24 (12): 619.

8. Macon-Lemaitre L, Triebel F. The negative regulatory function of the lymphocyte-activation gene-3 coreceptor (CD223) on human T cells. Immunology 2005; 115 (2): 170.

9. Kohler and Milstein. Continuous cultures of fused cells secreting antibody of predefined specificity. Nature. 1975; 256(5517): 495-497.

10. Kozbor et al., 1985, in Monoclonal Antibodies and Cancer Therapy, Alan R. Liss, Inc., pp. 77-96.

11. Ono et al, Improved technique of heart transplantation in rats. J. Thorac. Cardiovasc. Surg. 57, 225-229.

12. Stasiuk et al, Collagen-induced arthritis in DBA/1 mice: Cytokine gene activation following immunization with type II collagen. Cellular Immunol. 1996; 173: 269-275.

13. Campbell et al, Collagen-induced arthritis in C57BL/6 (H-2b) mice: new insights into an important disease model of rheumatoid arthritis. Eur. J. Immunol. 2000; 30: 1568-1575.

14. Inglis et al, Collagen-induced arthritis in C57BL/6 is associated with a robust and sustained T-cell response to type II collagen. Arthritis Research & Therapy. 2007; 9: R113.

15. Hiroaki et al, A tumor necrosis factor receptor loop peptide mimic inhibits bone destruction to the same extent as anti-tumor necrosis factor monoclonal ntibody in lurine collagen-induced arthritis. arthritis & Rheumatism. 2007; 56(4): 1164-1174.

**Claims**

1. A cytotoxic anti-LAG-3 monoclonal antibody or fragment thereof capable of causing depletion of LAG-3$^+$ activated T cells, wherein said antibody is produced by the hybridoma deposited at the CNCM on April 27, 2007 under the accession number CNCM I-3755, and further wherein said antibody or fragment thereof exhibits complement-dependent cytotoxicity (CDC) and/or antibody-dependent cell cytotoxicity (ADCC).

2. A cytotoxic anti-LAG-3 monoclonal antibody or fragment thereof capable of causing depletion of LAG-3$^+$ activated T cells, wherein said antibody is produced by the hybridoma deposited at the CNCM on April 27, 2007 under the accession number CNCM I-3756, and further wherein said antibody or fragment thereof exhibits complement-dependent cytotoxicity (CDC) and/or antibody-dependent cell cytotoxicity (ADCC).

3. A pharmaceutical composition comprising from 30 to 300 mg per dose of a cytotoxic anti-LAG-3 monoclonal antibody according to claim 1 or 2, and a pharmaceutically acceptable carrier and/or diluent for administration to a mammal.

4. A cytotoxic anti-LAG-3 monoclonal antibody or fragment thereof according to claim 1 or 2 for use in treating or preventing organ transplant rejection or for use in treating a T-cell mediated autoimmune disease.

**Patentansprüche**

1. Zytotoxischer monoklonaler anti-LAG-3-Antikörper oder Fragment davon, welcher/s fähig ist, Depletion von LAG-3$^+$ aktivierten T-Zellen zu verursachen, wobei der Antikörper durch das Hybridom produziert wird, welches bei der CNCM am 27. April 2007 unter der Hinterlegungsnummer CNCM I-3755 hinterlegt wurde, und ferner wobei der Antikörper oder das Fragment davon Komplement-abhängige Zytotoxizität (CDC) und/oder Antikörper-abhängige Zell-Zytotoxizität (ADCC) aufweist.

2. Zytotoxischer monoklonaler anti-LAG-3-Antikörper oder Fragment davon, welcher/s fähig ist, Depletion von LAG-3$^+$ aktivierten T-Zellen zu verursachen, wobei der Antikörper durch das Hybridom produziert wird, welches bei der CNCM am 27. April 2007 unter der Hinterlegungsnummer CNCM I-3756 hinterlegt wurde, und ferner wobei der Antikörper oder das Fragment davon Komplement-abhängige Zytotoxizität (CDC) und/oder Antikörper-abhängige Zell-Zytotoxizität (ADCC) aufweist.

3. Pharmazeutische Zusammensetzung, welche von 30 bis 300 mg pro Dosis eines zytotoxischen monoklonalen anti-LAG-3-Antikörpers gemäß Anspruch 1 oder 2 und ein/en pharmazeutisch akzeptablen/s Träger und/oder Verdünnungsmittel umfasst, zur Verabreichung an ein Säugetier.

4. Zytotoxischer monoklonaler Anti-LAG-3-Antikörper oder Fragment davon gemäß Anspruch 1 oder 2 zur Verwendung bei der Behandlung oder Prävention von Organtransplantatabstoßung oder zur Verwendung bei der Behandlung einer T-Zell-vermittelten Autoimmunerkrankung.

**Revendications**

1. Anticorps monoclonal cytotoxique anti-LAG-3 ou fragment de celui-ci apte à provoquer un appauvrissement de cellules T activées par LAG-3$^+$, dans lequel ledit anticorps est produit par l'hybridome déposé auprès du CNCM le 27 avril 2007 sous le numéro d'accès CNCM I-3755, et en outre dans lequel ledit anticorps ou fragment de celui-ci présente une cytotoxicité dépendant du complément (CDC) et/ou une cytotoxicité cellulaire dépendant de l'anticorps (ADCC).

2. Anticorps monoclonal cytotoxique anti-LAG-3 ou fragment de celui-ci apte à provoquer un appauvrissement de cellules T activées par LAG-3$^+$, dans lequel ledit anticorps est produit par l'hybridome déposé auprès du CNCM le 27 avril 2007 sous le numéro d'accès CNCM I-3756, et en outre dans lequel ledit anticorps ou fragment de celui-ci présente une cytotoxicité dépendant du complément (CDC) et/ou une cytotoxicité cellulaire dépendant de l'anticorps (ADCC).

3. Composition pharmaceutique comprenant de 30 à 300 mg par dose d'un anticorps monoclonal cytotoxique anti-LAG-3 selon la revendication 1 ou 2, et un vecteur et/ou un diluant pharmaceutiquement acceptables pour son administration à un mammifère.

4. Anticorps monoclonal cytotoxique anti-LAG-3 ou fragment de celui-ci selon la revendication 1 ou 2, pour son utilisation dans le traitement ou la prévention d'un rejet de transplantation d'organe ou pour son utilisation dans le traitement d'une maladie auto-immune médiée par cellules T.

Figure 1

Figure 2

Figure 3

Figure 4

Figure 5

Figure 6

**A**

**B**

**C**

**D**

**E**

**F**

Figure 7

**A**

**B**

Figure 8

D1 : Collagen (200µg) + CFA          D21 : Collagen (200µg) + CFA

Figure 9

Figure 10

**A**

ColE1 origin 4753...5473
pAS 4509...4744
gene hph 3474...4499
EM7 promoter 3421...3473
TK promoter 3279...3420
Matrix Attachment Region 3124...2568

pM-Hygro-SVPGK-
kappa huA9H12
chain
5476 bp

hGHpA 601...425
Kappa huA9H12 chain 1332...610
PGK promoter 2115...1345
SV40 enhancer 2321...2122
pAS 2487...2334

**B**

pUC origin 4762...5435
TKpA 4356...4626
Kana/Neo resistance 3386...4177
bla promoter 3332...3360
TK promoter 2845...3328
pAS 2835...2682

pTK-neo-SVPGK-
gamma huA9H12
chain
5438 bp

hGHpA 275...88
Gamma1 huA9H12 chain 1681...284
PGK promoter 2464...1694
SV40 enhancer 2670...2471

Figure 11

# EP 2 142 210 B1

Figure 12

Figure 13

Figure 14

Figure 15

# EP 2 142 210 B1

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2004078928 A
- WO 2005103086 A1
- US 4946778 A
- EP 07290545 A

### Non-patent literature cited in the description

- **MALGORZATA et al.** *European Journal of Ummunology,* 01 July 2005, vol. 35 (7), 2081-2088
- **WALDMANN H.** The new immunosuppression: just kill the T cell. *Nat Med,* 2003, vol. 9 (10), 1259
- **MONK NJ ; HARGREAVES RE ; MARSH JE et al.** Fc-dependent depletion of activated T cells occurs through CD40L-specific antibody rather than costimulation blockade. *Nat Med,* 2003, vol. 9 (10), 1275
- **ANDRE P ; PRASAD KS ; DENIS CV et al.** CD40L stabilizes arterial thrombi by a beta3 integrin--dependent mechanism. *Nat Med,* 2002, vol. 8 (3), 247
- **AVICE MN ; SARFATI M ; TRIEBEL F ; DELESPESSE G ; DEMEURE CE.** Lymphocyte activation gene-3, a MHC class II ligand expressed on activated T cells, stimulates TNF-alpha and IL-12 production by monocytes and dendritic cells. *J. Immunol.,* 1999, vol. 162, 2748
- **ANDREAE S ; PIRAS F ; BURDIN N ; TRIEBEL F.** Maturation and activation of dendritic cells induced by lymphocyte activation gene-3 (CD223). *J Immunol,* 2002, vol. 168 (8), 3874
- **ANDREAE S ; BUISSON S ; TRIEBEL F.** MHC class II signal transduction in human dendritic cells induced by a natural ligand, the LAG-3 protein (CD223). *Blood,* 2003, vol. 102 (6), 2130
- **TRIEBEL F.** LAG-3: a regulator of T-cell and DC responses and its use in therapeutic vaccination. *Trends Immunol,* 2003, vol. 24 (12), 619
- **MACON-LEMAITRE L ; TRIEBEL F.** The negative regulatory function of the lymphocyte-activation gene-3 coreceptor (CD223) on human T cells. *Immunology,* 2005, vol. 115 (2), 170
- **KOHLER ; MILSTEIN.** Continuous cultures of fused cells secreting antibody of predefined specificity. *Nature,* 1975, vol. 256 (5517), 495-497
- **KOZBOR et al.** Monoclonal Antibodies and Cancer Therapy. Alan R. Liss, Inc, 1985, 77-96
- **ONO et al.** Improved technique of heart transplantation in rats. *J. Thorac. Cardiovasc. Surg.,* vol. 57, 225-229
- **STASIUK et al.** Collagen-induced arthritis in DBA/1 mice: Cytokine gene activation following immunization with type II collagen. *Cellular Immunol.,* 1996, vol. 173, 269-275
- **CAMPBELL et al.** Collagen-induced arthritis in C57BL/6 (H-2b) mice: new insights into an important disease model of rheumatoid arthritis. *Eur. J. Immunol.,* 2000, vol. 30, 1568-1575
- **INGLIS et al.** Collagen-induced arthritis in C57BL/6 is associated with a robust and sustained T-cell response to type II collagen. *Arthritis Research & Therapy,* 2007, vol. 9, R113
- **HIROAKI et al.** A tumor necrosis factor receptor loop peptide mimic inhibits bone destruction to the same extent as anti-tumor necrosis factor monoclonal ntibody in lurine collagen-induced arthritis. *arthritis & Rheumatism,* 2007, vol. 56 (4), 1164-1174

SEQUENCE LISTING

<110> IMMUTEP
INSERM
TRIEBEL, Frédéric
VANHOVE, Bernard
HAUDEBOURG, Thomas

<120> CYTOTOXIC ANTI-LAG-3 MONOCLONAL ANTIBODY AND ITS USE IN THE TREATMENT OR PREVENTION OF ORGAN TRANSPLANT REJECTION AND AUTOIMMUNE DISEASE

<130> 49869/PCT

<150> PCT/IB2008/XXXXX
<151> 2008-04-30

<150> EP 07290545.8
<151> 2007-04-30

<160> 7

<170> PatentIn version 3.3

<210> 1
<211> 25
<212> PRT
<213> Rattus norvegicus

<400> 1

```
Asp Gln Pro Ala Ser Ile Pro Ala Leu Asp Leu Leu Gln Gly Met Pro
1               5                   10                  15


Ser Thr Arg Arg His Pro Pro His Arg
            20                  25
```

<210> 2
<211> 28
<212> DNA
<213> Artificial

<220>
<223> Primer

<400> 2
atatgaattc acagaggaga tgaggcag 28

<210> 3
<211> 28
<212> DNA
<213> Artificial

<220>
<223> Primer

<400> 3
atatgaattc tcctggtcag agctgcct 28

<210> 4
<211> 21
<212> DNA
<213> Artificial

<220>
<223> Primer

<400> 4
tggatgctat ggaaggaaag a         21

<210> 5
<211> 20
<212> DNA
<213> Artificial

<220>
<223> Primer

<400> 5
gattctggtg acagctggtg         20

<210> 6
<211> 21
<212> DNA
<213> Artificial

<220>
<223> Primer

<400> 6
ccttggtcaa gcagtacagc c         21

<210> 7
<211> 22
<212> DNA
<213> Artificial

<220>
<223> Primer

<400> 7
ttcgctgatg acacaaacat ga         22